# EUROPEAN PATENT APPLICATION

(11) **EP 4 364 844 A1**
(43) Date of publication of application: **08.05.2024**
(21) Application number: 22833332.4
(22) Date of filing: 01.07.2022
(51) Int. Cl.: B01J 29/46, B01J 37/16, C10L 3/12

(54) **CATALYST FOR SYNTHESIZING LIQUEFIED PETROLEUM GAS AND METHOD FOR PRODUCING LIQUEFIED PETROLEUM GAS**

(30) Priority: 02.07.2021 JP 2021111072; 15.04.2022 JP 2022067955
(71) Applicant: FURUKAWA ELECTRIC CO., LTD., Tokyo 100-8322 (JP)
(72) Inventor: FUJIKAWA Takashi, Tokyo 100-8322 (JP); IWANO Yuki, Tokyo 100-8322 (JP); MORI Tomohiko, Tokyo 100-8322 (JP); TAKAHASHI Hiroko, Tokyo 100-8322 (JP); BAMBA Yuichiro, Tokyo 100-8322 (JP); KAWAMATA Yuki, Tokyo 100-8322 (JP); LEE Yu, Tokyo 100-8322 (JP); HIRANO Junya, Tokyo 100-8322 (JP); FUKUSHIMA Masayuki, Tokyo 100-8322 (JP)
(74) Representative: Winter, Brandl - Partnerschaft mbB
(86) International application number: PCT/JP2022/026507
(87) International publication number: WO 2023/277187

(57) **Abstract**

A catalyst for synthesizing liquefied petroleum gas according to the present invention includes: a Cu-Zn-based catalytic material; and an MFI-type zeolite catalytic material supporting Pt, in which a ratio (M1/(M1+M2)) of mass (M1) of the Cu-Zn-based catalytic material to total mass of the mass (M1) of the Cu-Zn-based catalytic material and mass (M2) of the MFI-type zeolite catalytic material is 0.30 or more and 0.95 or less.

## Description

### TECHNICAL FIELD

The present disclosure relates to a catalyst for synthesizing liquefied petroleum gas and a method for producing liquefied petroleum gas.

### BACKGROUND ART

Liquefied petroleum gas (LPG) mainly consisting of propane and butane exists mixed with impurity gases such as methane and ethane in oil fields and natural gas fields. After transferring such gases to aboveground facilities, propane and butane are separated and recovered from the gases, further, impurities such as sulfur and mercury are removed to obtain liquefied petroleum gas.

Additionally, liquefied petroleum gas is also contained in crude oil. Therefore, it is also possible to obtain liquefied petroleum gas by separating and extracting propane and butane during the refining process at oil refineries.

Moreover, methods for producing liquefied petroleum gas from synthesis gas of carbon monoxide and hydrogen have been studied. For instance, Patent Documents 1 to 4 disclose methods for producing liquefied petroleum gas in which the main component is butane.

Non-Patent Documents 1 and 2 disclose methods for producing hydrocarbons including isobutane as the main component from the synthesis gas of carbon monoxide and hydrogen.

In the aforementioned patent documents, liquefied petroleum gas and gasoline are primarily produced using a mixed catalyst of a methanol synthesis catalyst and zeolite supporting palladium.

In the synthesis of liquefied petroleum gas using conventional mixed catalysts as disclosed in the above patent documents, when synthesis is conducted at high temperature to enhance the yield of liquefied petroleum gas such as propane and butane, the mixed catalyst deactivates, causing a decline in the yield of liquefied petroleum gas over time; therefore, consistently synthesizing liquefied petroleum gas over a long period of time may become difficult. In particular, when using Cu-Zn-based catalysts with good catalytic activity, deactivation due to high temperature is pronounced.

On the other hand, to suppress such catalyst deactivation due to high temperature, when synthesizing liquefied petroleum gas at low temperature using conventional mixed catalysts as disclosed in the aforementioned patent documents, there is a problem that the yield of liquefied petroleum gas such as propane is extremely low.

Moreover, propane is easily vaporized and is therefore preferable as a fuel even in cold regions; therefore, among liquefied petroleum gases, it is desirable to have a particularly high yield of propane.

### Citation List

### Patent Document

Patent Document 1: Japanese Patent No. 5405103
Patent Document 2: Japanese Patent No. 4965258
Patent Document 3: Japanese Patent No. 4558751
Patent Document 4: Japanese Unexamined Patent Application, Publication No. 2019-37939

### Non-Patent Document

Non-Patent Document 1: Congming Li, Kaoru Fujimoto, "Synthesis gas conversion to iso-butane-rich hydrocarbons over a hybrid catalyst containing Beta zeolite - role of doped palladium and influence of the SiO2/Al2O3 ratio", Catalysis Science & Technology, 2015, 5, 4501-4510
Non-Patent Document 2: Congming Li, Hongyan Ban, Weijie Cai, Yu Zhang, Zhong Li, Kaoru Fujimoto, "Direct synthesis of iso-butane from synthesis gas or CO2 over CuZnZrAl/Pd-β hybrid catalyst", Journal of Saudi Chemical Society (2017)21, 974-982

### DISCLOSURE OF THE INVENTION

### Problems to be Solved by the Invention

The object of the present disclosure is to provide a catalyst for synthesizing liquefied petroleum gas and a method for producing liquefied petroleum gas, which can produce propane with a high yield even at low synthesis temperature of liquefied petroleum gas.

### Means for Solving the Problems

[1] A catalyst for synthesizing liquefied petroleum gas including a Cu-Zn-based catalytic material and an MFI-type zeolite catalytic material supporting Pt, in which a ratio (M1/(M1+M2)) of mass (M1) of the Cu-Zn-based catalytic material to total mass of the mass (M1) of the Cu-Zn-based catalytic material and mass (M2) of the MFI-type zeolite catalytic material is 0.30 or more and 0.95 or less.
[2] The catalyst for synthesizing liquefied petroleum gas as described in [1], in which the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material is 0.30 or more and less than 0.70.
[3] The catalyst for synthesizing liquefied petroleum gas as described in [1], in which the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material is 0.50 or more and 0.95 or less.
[4] The catalyst for synthesizing liquefied petroleum gas as described in any one of [1] to [3], in which the MFI-type zeolite catalytic material supports only Pt.
[5] The catalyst for synthesizing liquefied petroleum gas as described in any one of [1] to [3], in which the MFI-type zeolite catalytic material further supports Pd.
[6] The catalyst for synthesizing liquefied petroleum gas as described in [5], in which a ratio (M_{Pd}/(M_{Pt}+M_{Pd})) of mass (M_{pd}) of Pd to total mass (M_{Pt}+M_{Pd}) of mass (M_{Pt}) of Pt and the mass (M_{Pd}) of Pd supported on the MFI-type zeolite catalytic material is 0.70 or less.
[7] The catalyst for synthesizing liquefied petroleum gas as described in [5] or [6], in which a ratio of the total mass (M_{Pt}+M_{Pd}) of the mass (M_{Pt}) of Pt and the mass (M_{pd}) of Pd in the MFI-type zeolite catalytic material to the mass (M2) of the MFI-type zeolite catalytic material is 0.1 mass% or more and 1.0 mass% or less.
[8] The catalyst for synthesizing liquefied petroleum gas as described in any one of [1] to [7], in which the Cu-Zn-based catalytic material and the MFI-type zeolite catalytic material exist independently from each other, and both of the Cu-Zn-based catalytic material and the MFI-type zeolite catalytic material are in the form of granulated powder or molded body.
[9] A method for producing liquefied petroleum gas including: a reduction treatment step of reducing the catalyst for synthesizing liquefied petroleum gas as described in any one of [1] to [8]; a supply step of supplying carbon monoxide and hydrogen to the catalyst for synthesizing liquefied petroleum gas reduced in the reduction treatment step; and a synthesis step of synthesizing liquefied petroleum gas by reacting the carbon monoxide and the hydrogen supplied in the supply step using the catalyst for synthesizing liquefied petroleum gas to be reduced.
[10] The method for producing liquefied petroleum gas as described in [9], in which a gas hourly space velocity (GHSV) for supplying the carbon monoxide and the hydrogen is 300/h or more and 20000/h or less in the supply step.
[11] The method for producing liquefied petroleum gas as described in [9] or [10], in which the carbon monoxide and the hydrogen are reacted at a temperature of 260°C or more and 360°C or less in the synthesis step.
[12] The method for producing liquefied petroleum gas as described in any one of [9] to [11], in which the carbon monoxide and the hydrogen are reacted under a pressure of 2.0 MPa or more and 6.0 MPa or less in the synthesis step.

### Effects of the Invention

According to the present disclosure, it is possible to provide a catalyst for synthesizing liquefied petroleum gas and a method for producing liquefied petroleum gas, which can produce propane with a high yield even at low synthesis temperature of liquefied petroleum gas.

### BRIEF DESCRIPTION OF THE DRAWINGS

Fig. 1 is a diagram illustrating the results of Examples 1 to 3 and Comparative Example 1.
Fig. 2 is a diagram illustrating the results of Examples 3 to 5.
Fig. 3 is a diagram illustrating the results of Comparative Examples 2 to 5.
Fig. 4 is a diagram illustrating the mid-term stability test results of Example 10.
Fig. 5 is a diagram illustrating the long-term stability test results of Example 8.

### PREFERRED MODE FOR CARRYING OUT THE INVENTION

A detailed description based on the embodiments follows.

The present inventors, after intensive research, found that in a catalyst for synthesizing liquefied petroleum gas including a Cu-Zn-based catalytic material and a zeolite catalytic material, by specifying the ratio (M1/(M1+M2)) of the mass (M1) of the Cu-Zn-based catalytic material to the total mass of the mass (M1) of the Cu-Zn-based catalytic material and the mass (M2) of the zeolite catalytic material within a particular range, and specifying the composition and supported material of the zeolite catalytic material, it is possible to produce propane with a high yield even at low synthesis temperature of liquefied petroleum gas. Such findings led to the completion of the present disclosure.

The catalyst for synthesizing liquefied petroleum gas of the present embodiment includes a Cu-Zn-based catalytic material and an MFI-type zeolite catalytic material (hereinafter, also simply referred to as the zeolite catalytic material) supporting Pt, in which the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material is 0.30 or more and 0.95 or less.

As mentioned above, the catalyst for synthesizing liquefied petroleum gas of the embodiment includes a Cu-Zn-based catalytic material and an MFI-type zeolite catalytic material. The catalyst for synthesizing liquefied petroleum gas can synthesize liquefied petroleum gas from carbon monoxide and hydrogen. The liquefied petroleum gas synthesized by the catalyst for synthesizing liquefied petroleum gas of the present embodiment mainly consists of propane and butane, with propane being more predominant than butane. In the liquefied petroleum gas synthesized by the catalyst for synthesizing liquefied petroleum gas of the present embodiment, the ratio of the combined total of propane and butane to the liquefied petroleum gas is, for example, 20 Cmol% or more. Moreover, the ratio of propane to the combined total of propane and butane in the liquefied petroleum gas synthesized by the catalyst for synthesizing liquefied petroleum gas of the present embodiment is, for example, 55 vol% or more.

The Cu-Zn-based catalytic material composing the catalyst for synthesizing liquefied petroleum gas has the function as liquefied petroleum gas precursor synthesis catalyst to synthesize the liquefied petroleum gas precursors such as methanol and dimethyl ether from carbon monoxide and hydrogen.

The Cu-Zn-based catalytic material composing the catalyst for synthesizing liquefied petroleum gas is a catalyst including copper oxide and zinc oxide, and excels in performance of synthesizing liquefied petroleum gas precursors, among catalysts for synthesizing liquefied petroleum gas precursors. The Cu-Zn-based catalytic material may also include, in addition to copper oxide and zinc oxide, other component such as aluminum oxide, gallium oxide, zirconium oxide, or indium oxide. By including component such as aluminum oxide, gallium oxide, zirconium oxide, or indium oxide, the dispersion of copper oxide and zinc oxide can be improved. From the perspective of efficiently forming the number of interfaces between copper and zinc, which are believed to be active sites, it is preferable that the Cu-Zn-based catalytic material is a ternary oxide consisting of copper oxide, zinc oxide, and aluminum oxide.

The zeolite catalytic material composing the catalyst for synthesizing liquefied petroleum gas synthesizes liquefied petroleum gas from the liquefied petroleum gas precursors generated by the Cu-Zn-based catalytic material. In the present embodiment, the synthesized liquefied petroleum gas contains propane and butane as its primary components, with propane being more predominant than butane.

Regarding the zeolite catalytic material that composes the catalyst for synthesizing liquefied petroleum gas, the type of zeolite is MFI-type. The MFI-type zeolite catalytic material has a smaller pore diameter compared to beta-type zeolite, and it is assumed that propane can be synthesized more efficiently than butane among components of liquefied petroleum gas and the yield of propane can be enhanced.

Also, the MFI-type zeolite catalytic material supports Pt (platinum). Preferably, the MFI-type zeolite catalytic material supports only Pt. The MFI-type zeolite catalytic material supporting Pt can efficiently react the liquefied petroleum gas precursors, potentially leading to a higher yield of propane.

In the present embodiment, the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material (hereinafter also simply referred to as the mass ratio of the Cu-Zn-based catalytic material) is 0.30 or more and 0.95 or less. When the mass ratio of the Cu-Zn-based catalytic material falls within the aforementioned range, liquefied petroleum gas can be efficiently synthesized from carbon monoxide and hydrogen.

Within the aforementioned range, when the ratio (M1/(M1+M2)) is 0.30 or more and less than 0.70, the initial catalytic activity of the catalyst for synthesizing liquefied petroleum gas is high. For this ratio, the lower limit is preferably 0.35 or more, and more preferably 0.40 or more, while the upper limit is preferably 0.65 or less, and more preferably 0.60 or less.

Additionally, within the aforementioned range, when the ratio (M1/(M1+M2)) is 0.50 or more and 0.95 or less, the catalyst for synthesizing liquefied petroleum gas demonstrates superior long-term stability. For this ratio, the lower limit is preferably 0.50 or more, more preferably 0.60 or more, and even more preferably 0.70 or more, while the upper limit is preferably 0.95 or less, more preferably 0.90 or less, and even more preferably 0.85 or less.

The reaction of producing liquefied petroleum gas from CO and H₂ on the catalyst for synthesizing liquefied petroleum gas progresses with the following reaction mechanism. Initially, CO and H₂ are converted to methanol that is a liquefied petroleum gas precursor on the Cu-Zn-based catalytic material (for example, CuZnOZrO₂Al₂O₃). Subsequently, the produced methanol undergoes dehydration at acid sites on an MFI-type zeolite catalyst (for example, Pt/P-ZSM-5) to convert to dimethyl ether. Dimethyl ether then undergoes further dehydration at the acid sites on the MFI-type zeolite catalyst, cycling through carbon increasing reactions and decomposing reactions, and transforming into olefins. These generated olefins are then hydrogenated on noble metals such as Pt on the MFI-type zeolite catalyst, leading to the production of liquefied petroleum gas and the like.

In synthesizing liquefied petroleum gas, raising the synthesis temperature may increase the yield of liquefied petroleum gas such as propane. However, when using the Cu-Zn-based catalyst having superior performance in synthesizing liquefied petroleum gas precursors, there is a tendency to aggregate at higher temperature, causing notable temporal deactivation of the Cu-Zn-based catalyst, making stable synthesis of liquefied petroleum gas over extended periods difficulty. On the other hand, when the synthesis temperature is reduced (for example, to 330°C or below) to suppress the deactivation of the Cu-Zn-based catalyst due to such high temperature, the deactivation of the Cu-Zn-based catalyst can be suppressed, but the yield of liquefied petroleum gas such as propane will be reduced.

Nevertheless, by using the catalyst for synthesizing liquefied petroleum gas of the present embodiment, it is possible to produce propane with high yield even at lower synthesis temperature (for instance, at 330°C or below) of the liquefied petroleum gas. Therefore, by using the catalyst for synthesizing liquefied petroleum gas of the present embodiment and synthesizing at lower temperature (for instance, at 330°C or below), propane can be produced at high yield, while also suppressing the deactivation of the catalyst due to high temperature. The synthesis temperature refers to the temperature of the catalyst for synthesizing liquefied petroleum gas.

In the present embodiment, even at low synthesis temperature (for example, at 330°C or below), the yield of propane in the produced liquefied petroleum gas is for example 10 Cmol% or higher, and can be increased to 15 Cmol% or higher or even to 17 Cmol% or higher.

By using the catalyst for synthesizing liquefied petroleum gas of the present embodiment, a higher yield of butane can also be achieved. For example, even at lower synthesis temperature (for example, at 330°C or below), the yield of butane is for example 8 Cmol% or higher, and can be increased to 10 Cmol% or higher or even to 12 Cmol% or higher.

Furthermore, in the present embodiment, the yield of propane and butane (the combined total of the yield of propane and the yield of butane) is, for example, 25 Cmol% or higher and preferably 30 Cmol% or higher.

By using the catalyst for synthesizing liquefied petroleum gas of the present embodiment, the ratio of propane to the combined total of propane and butane can be increased. In the liquefied petroleum gas produced in the present embodiment, the ratio of propane to the combined total of propane and butane ((molar number of propane / (molar number of propane + molar number of butane)) × 100) is, for example, 55 vol% or more, preferably 60 vol% or more, more preferably 65 vol% or more, and even more preferably 70 vol%.

Moreover, the zeolite catalytic material may also support platinum group element such as Pd (palladium), Rh (rhodium) or Ru (ruthenium) in addition to Pt. The noble metal can be single type or plural type. When the noble metal is plural type, there is no specific restriction on the state of the noble metals supported on the zeolite catalytic material; for example, the noble metals may coexist as individual metals, form alloy, or coexist as individual metals and alloy.

Among them, the zeolite catalytic material preferably supports Pd (palladium) in addition to Pt. By supporting Pd in addition to Pt, the yield of butane can be increased while maintaining a high yield of propane as in the case of supporting only Pt.

Regarding the state of Pt and Pd supported on the zeolite catalytic material, individual metal Pt and individual metal Pd may coexist, Pt and Pd may form an alloy, or at least one of the individual metals of Pt and Pd and an alloy of Pt and Pd may coexist.

When the zeolite catalytic material supports Pt and Pd, regarding the ratio (M_{Pd}/(M_{Pt}+M_{Pd})) of the mass (M_{Pd}) of Pd to the total mass (M_{Pt}+M_{Pd}) of mass (M_{Pt}) of Pt and the mass (M_{pd}) of Pd supported on the zeolite catalytic material (hereinafter also referred to as mass ratio (M_{Pd}/(M_{Pt}+M_{Pd}))), the upper limit is preferably 0.70 or less, more preferably 0.60 or less, and even more preferably 0.50 or less. The lower limit of the mass ratio (M_{Pd}/(M_{Pt}+M_{Pd})) is, for instance, 0.01 or more, preferably 0.15 or more, more preferably 0.20 or more, and even more preferably 0.25 or more. When the mass ratio (M_{Pd}/(M_{Pt}+M_{Pd})) is 0.70 or less, the yield of butane can be further increased.

Additionally, regarding the ratio of the total mass (M_{Pt}+M_{Pd}) of the mass (M_{Pt}) of Pt and the mass (M_{Pd}) of Pd in the zeolite catalytic material to the mass (M2) of the zeolite catalytic material, the lower limit is preferably 0.1 mass% or more, more preferably 0.2 mass% or more, and even more preferably 0.3 mass% or more, and the upper limit is preferably 1.0 mass% or less, more preferably 0.8 mass% or less, and even more preferably 0.7 mass% or less. When the zeolite catalytic material does not support Pd, the content ratio of Pd in the total content ratio is 0 (zero), which means that the total content ratio represents the content ratio of Pt.

When the ratio of the total mass (M_{Pt}+M_{Pd}) of the mass (M_{Pt}) of Pt and the mass (M_{pd}) of Pd to the mass (M2) of the zeolite catalytic material is 0.1 mass% or more, liquefied petroleum gas can be synthesized efficiently. Moreover, when the ratio of the total mass (M_{Pt}+M_{Pd}) of the mass (M_{Pt}) of Pt and the mass (M_{pd}) of Pd to the mass (M2) of the zeolite catalytic material is 1.0 mass% or less, efficient synthesis of liquefied petroleum gas can be maintained while suppressing the increase in material costs of Pt and Pd.

The presence or absence of Pt and Pd supported on the zeolite catalytic material, the mass ratio (M_{Pd}/(M_{Pt}+M_{Pd})), and the ratio of the total mass (M_{Pt}+M_{Pd}) of the mass (M_{Pt}) of Pt and the mass (M_{Pd}) of Pd to the mass (M2) of the zeolite catalytic material can be measured by ICP-OES (Inductively Coupled Plasma Optical Emission Spectroscopy).

Additionally, in the zeolite catalytic material, the ratio of the molar number of SiO₂ to the molar number of Al₂O₃ (molar number of SiO₂ / molar number of Al₂O₃) (hereinafter simply referred to as the "molar ratio (SiO₂/Al₂O₃)") is preferably 20 or more and 60 or less. The zeolite catalytic material is an aluminosilicate. By substituting some of the silicon atoms in the silicate forming the zeolite framework of the zeolite catalytic material with aluminum atoms, the aluminum atoms become acid sites; therefore, the zeolite catalytic material exhibits functionality as a solid acid.

When the molar ratio (SiO₂/Al₂O₃) is 60 or less, the acid sites of the zeolite catalytic material increase; this leads to an increase in the production amount of liquefied petroleum gas and also to an increase in the amount of propane contained in the liquefied petroleum gas due to efficient synthesis of propane. Also, when the molar ratio (SiO₂/Al₂O₃) is 20 or more, the zeolite catalytic material supporting Pt can be produced easily while maintaining a high liquefied petroleum gas production capability and a high propane synthesis capability.

From the perspective of ease of production of the zeolite catalytic material, the molar ratio (SiO₂/Al₂O₃) is preferably 20 or more, more preferably 25 or more, and even more preferably 30 or more. From the perspective of high catalytic performance, the molar ratio (SiO₂/Al₂O₃) is preferably 60 or less, more preferably 50 or less, and even more preferably 40 or less.

The molar ratio (SiO₂/Al₂O₃) can be measured by ICP-OES (Inductively Coupled Plasma Optical Emission Spectroscopy).

Furthermore, the solid acid amount of the zeolite catalytic material is preferably 0.6 mmol/g or more, more preferably 0.8 mmol/g or more. When the solid acid amount is 0.6 mmol/g or more, the zeolite catalytic material supporting the noble metal can be produced easily while maintaining the high liquefied petroleum gas production capability and the high propane synthesis capability.

The above-mentioned solid acid amount can be measured by NH₃-TPD (Ammonia Temperature-Programmed Desorption).

The Cu-Zn-based catalytic material and the MFI-type zeolite catalytic material preferably exist independently from each other, and both of the Cu-Zn-based catalytic material and the MFI-type zeolite catalytic material are preferably in the form of powder or molded body. For the catalyst for synthesizing liquefied petroleum gas, the Cu-Zn-based catalytic material and the MFI-type zeolite catalytic material are preferably not integrated (indistinctly integrated). The state of the Cu-Zn-based catalytic material and the zeolite catalytic material may be in the form of granulated powder (powder. for example, particle size of 10⁻⁹ to 10⁻⁴ m) or granules having a particle size larger than the granulated powder; however, when the Cu-Zn-based catalytic material is a molded body containing Cu-Zn-based catalytic material, and the zeolite catalytic material is a molded body containing zeolite catalytic material, the yield of propane and butane can be further increased. In other words, the catalyst for synthesizing liquefied petroleum gas is preferably a mixture of the molded bodies containing the Cu-Zn-based catalytic material and the molded bodies containing the zeolite catalytic material.

For the molded body containing the Cu-Zn-based catalytic material, the content ratio of the Cu-Zn-based catalytic material contained in the molded body is preferably 80 mass% or more, more preferably 90 mass% or more, and even more preferably 95 mass% or more. When this content ratio is 80 mass% or more, the liquefied petroleum gas precursors can be efficiently synthesized from carbon monoxide and hydrogen. The content ratio of the Cu-Zn-based catalytic material contained in the molded body may be 100 mass%. Additionally, this content ratio is preferably 98 mass% or less, more preferably 96 mass% or less, and even more preferably 94 mass% or less. When this content ratio is 98 mass% or less, the moldability and the mechanical strength of the molded body can be improved while maintaining efficient synthesis of the liquefied petroleum gas precursors.

The molded body containing the Cu-Zn-based catalytic material may also include various additives to improve the moldability and the mechanical strength, in addition to the Cu-Zn-based catalytic material. Examples of such additives include molding binders such as graphite and carbon black.

For the molded body containing the zeolite catalytic material, the content ratio of the zeolite catalytic material contained in the molded body is preferably 70 mass% or more, more preferably 80 mass% or more, and even more preferably 90 mass% or more. When this content ratio is 70 mass% or more, liquefied petroleum gas can be efficiently synthesized from the liquefied petroleum gas precursors. The content ratio of the zeolite catalytic material contained in the molded body may be 100 mass%. Additionally, this content ratio is preferably 98 mass% or less, more preferably 96 mass% or less, and even more preferably 94 mass% or less. When this content ratio is 98 mass% or less, the moldability and the mechanical strength of the molded body can be improved while maintaining efficient synthesis of liquefied petroleum gas.

Similar to the molded body containing the Cu-Zn-based catalytic material, the molded body containing the zeolite catalytic material may also include various additives to improve the moldability and the mechanical strength, in addition to the zeolite catalytic material. Examples of such additives include molding binders such as various clay binders, alumina-based binder, and silica-based binder. Various clay binders include kaolin-based binder, bentonite-based binder, talc-based binder, pyrophyllite-based binder, molysite-based binder, vermiculite-based binder, montmorillonite-based binder, chlorite-based binder, halloysite-based binder, etc. To effectively improve the catalyst activity and suppress the formation of catalyst poisoning substance such as coke, the molding binder is preferably a silica-based binder.

The shape of the Cu-Zn-based catalytic material and the zeolite catalytic material is not limited in particular. When the Cu-Zn-based catalytic material or the zeolite catalytic material is in a molded body, shapes such as cylindrical, clover-like, ring-like, spherical, or multi-holed can be chosen, for instance. In the case of cylindrical or clover-like shaped molded body, such molded body is preferably an extrusion-molded body.

Regarding the particle size of the molded body containing the Cu-Zn-based catalytic material and the molded body containing the zeolite catalytic material, the lower limit is preferably 200 um or more, more preferably 300 um or more, and the upper limit is preferably 10 mm or less, more preferably 5 mm or less, and even more preferably 3 mm or less. When such particle size is 200 um or more, pressure loss within the reactor can be prevented. When such particle size is 10 mm or less, the contact efficiency between the reactant and the catalyst in the reactor can be enhanced. The particle size can be determined by the dry sieving test method.

Furthermore, concerning the bulk density of the molded body containing the Cu-Zn-based catalytic material and the molded body containing the zeolite catalytic material, the lower limit is preferably 0.5 g/cm³ or more, and the upper limit is preferably 1.5 g/cm³ or less, more preferably 1.0 g/cm³ or less. The bulk density can be determined using the sock loading bulk density measurement method with a measuring cylinder.

Moreover, the zeolite catalytic material preferably further contains P (phosphorus). When the zeolite catalytic material contains P, the acid sites (solid acid sites) of the zeolite catalytic material increase and simultaneously shift to weaker acid sites, thereby allowing for increasing the ratio of propane to the combined total of propane and butane. When the zeolite catalytic material contains P, it is presumed, as shown in the following formula (1), that P binds to the oxygen (O) associated with Si and the oxygen (O) associated with Al present on the surface of the zeolite catalytic material.

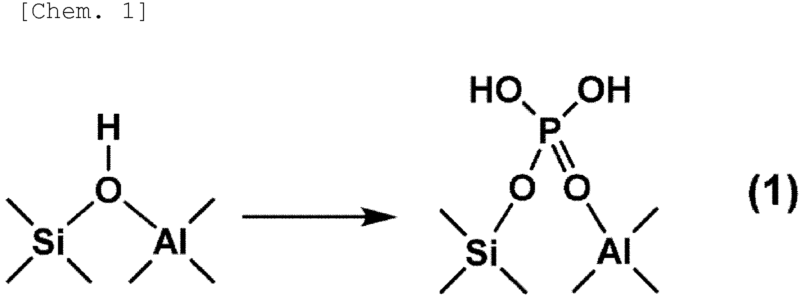

For the content ratio of P contained in the zeolite catalytic material, regarding the ratio of the mass (M_{P}) of P in the zeolite catalytic material to the mass (M2) of the zeolite catalytic material, the lower limit is preferably more than 0 mass%, more preferably 0.5 mass% or more, even more preferably 1.0 mass% or more, and especially preferably 1.5 mass% or more, and the upper limit is preferably less than 5.0 mass%, more preferably 4.0 mass% or less, even more preferably 3.0 mass% or less, and especially preferably 2.5 mass% or less.

When the ratio of the mass (M_{P}) of P to the mass (M2) of the zeolite catalytic material is more than 0 mass%, it is possible to increase the ratio of propane to the combined total of propane and butane. Furthermore, when the ratio of the mass (M_{P}) of P to the mass (M2) of the zeolite catalytic material is less than 5.0 mass%, it is possible to prevent the decrease in the ratio of propane to the combined total of propane and butane and the decrease in the yield of propane and butane due to excessive content of P.

The presence or absence of P in the zeolite catalytic material, and the content ratio of P can be measured by ICP-OES (Inductively Coupled Plasma Optical Emission Spectroscopy).

Elements that function similarly to P include B, Mg, Ca, La, and Zr. These elements can change the acidic nature of the zeolite catalytic material from strong acid to weak acid, just like P. Therefore, when the zeolite catalytic material contains B, Mg, Ca, La, or Zr in the same amount as the aforementioned P, the ratio of propane to the combined total of propane and butane will increase.

Next, the method for producing liquefied petroleum gas in the embodiment will be described.

The method for producing liquefied petroleum gas in the embodiment includes a reduction treatment step, a supply step, and a synthesis step.

In the reduction treatment step, the catalyst for synthesizing liquefied petroleum gas is reduced. Preferably, the catalyst for synthesizing liquefied petroleum gas is reduced with hydrogen.

In the supply step, performed after the reduction treatment step, carbon monoxide (CO) and hydrogen (H₂) are supplied to the catalyst for synthesizing liquefied petroleum gas reduced in the reduction treatment step. Carbon monoxide and hydrogen are both in gaseous form. Regarding the supply method, carbon monoxide and hydrogen may be supplied separately, or a mixed gas containing carbon monoxide and hydrogen, such as synthesis gas, may be supplied.

In the synthesis step, performed after the supply step, carbon monoxide and hydrogen supplied in the supply step are reacted using the reduced catalyst for synthesizing liquefied petroleum gas to produce liquefied petroleum gas. By using the catalyst for synthesizing liquefied petroleum gas to react carbon monoxide and hydrogen, even when the synthesis temperature (temperature of the catalyst in the synthesis step) is low, for instance, 330°C or below, it is possible to achieve a high yield of propane. Additionally, the catalyst for synthesizing liquefied petroleum gas of the present embodiment is resistant to deactivation, has excellent long-term stability, and can maintain good catalytic performance for a long period (for example, 70 hours or longer when the synthesis temperature is 330°C or below).

Regarding the gas hourly space velocity (GHSV) for supplying carbon monoxide and hydrogen in the supply step, the lower limit is preferably 300/h or more, more preferably 500/h or more, and even more preferably 1000/h or more; the upper limit is preferably 20000/h or less, more preferably 10000/h or less, and even more preferably 5000/h or less.

When the gas hourly space velocity (GHSV) is 300/h or more, it is possible to efficiently produce liquefied petroleum gas from carbon monoxide and hydrogen. Additionally, when the gas hourly space velocity (GHSV) is 20000/h or less, it is possible to suppress the increase in the content ratio of unreacted substance such as carbon monoxide or hydrogen in the gas containing the produced liquefied petroleum gas obtained after synthesis.

Regarding the temperature of the catalyst (synthesis temperature) in the synthesis step, the lower limit is preferably 260°C or more, more preferably 270°C or more, and even more preferably 280°C or more; the upper limit is preferably 360°C or less, more preferably 340°C or less, even more preferably 330°C or less, and especially preferably 320°C or less.

In the synthesis step, by reacting carbon monoxide and hydrogen at a temperature of 260°C or more, it is possible to efficiently produce liquefied petroleum gas from carbon monoxide and hydrogen. Additionally, by reacting carbon monoxide and hydrogen at a temperature of 360°C or less in the synthesis step, it is possible to suppress the deterioration of the catalytic performance of the catalyst for synthesizing liquefied petroleum gas due to temperature. Also, by reacting carbon monoxide and hydrogen at a temperature of 360°C or less, it is possible to suppress the decrease in the yield due to excessive decomposition of the produced liquefied petroleum gas (e.g., decomposition from propane to ethane, or from ethane to methane).

Regarding the pressure in the synthesis step, the lower limit is preferably 2.0 MPa or more, more preferably 3.0 MPa or more, and even more preferably 3.5 MPa or more; the upper limit is preferably 6.0 MPa or less, more preferably 5.5 MPa or less, and even more preferably 5.0 MPa or less, under which carbon monoxide and hydrogen are reacted.

In the synthesis step, by reacting carbon monoxide and hydrogen at a pressure of 2.0 MPa or more, it is possible to efficiently produce liquefied petroleum gas from carbon monoxide and hydrogen. Furthermore, by reacting carbon monoxide and hydrogen at a pressure of 6.0 MPa or less in the synthesis step, it is possible to suppress the deactivation of the catalytic performance of the catalyst for synthesizing liquefied petroleum gas due to pressure.

The catalyst for synthesizing liquefied petroleum gas can be produced, for instance, by mixing the Cu-Zn-based catalytic material and the zeolite catalytic material. The composition, ratio, and state of the Cu-Zn-based catalytic material and the zeolite catalytic material can be appropriately set depending on the desired liquefied petroleum gas.

The aforementioned molar ratio (SiO₂/Al₂O₃) of the zeolite catalytic material can be controlled by the amount of aluminum source added during the synthesis of the zeolite catalytic material.

The amount of solid acid in the zeolite catalytic material can be controlled by, for example, the synthesis conditions (such as pH) during the synthesis of the zeolite catalytic material.

The method for supporting noble metal such as platinum or palladium on the zeolite catalytic material is not particularly limited, but such method includes the impregnation method, immersion method, and ion-exchange method.

When supporting platinum and palladium on the zeolite catalytic material, it is preferable to simultaneously support both platinum and palladium using an impregnating solution or an immersion solution containing platinum and palladium, rather than sequentially supporting each metal through separate impregnation or immersion.

A compound containing platinum or palladium can be used as a starting material for platinum or palladium to be supported on the zeolite catalytic material. For the starting material for platinum, chloroplatinic acid hexahydrate, dinitrodiamine platinum, dichlorotetraamine platinum, platinum oxide, and platinum chloride can be used. For the starting material for palladium, palladium chloride, palladium nitrate, dinitrodiamine palladium, palladium sulfate, and palladium oxide can be used.

After impregnating the zeolite catalytic material with a solution of a compound containing platinum or palladium, such as a chloroplatinic acid solution or a palladium chloride solution, or after immersing the zeolite catalytic material in the solution of the compound containing platinum or palladium, calcining the impregnated or immersed zeolite catalytic material can efficiently highly disperse Pt or Pd in the zeolite catalytic material and easily control the amount of Pt or Pd supported on the zeolite catalytic material.

The concentration of the compound containing platinum or palladium in the solution may be set according to the amount of platinum or palladium to be supported. For instance, the concentration of the chloroplatinic acid hexahydrate solution is preferably 0.15 mass% or more and 3.50 mass% or less. The concentration of the palladium chloride solution is preferably 0.1 mass% or more and 2.5 mass% or less. The supporting amount of Pt or Pd can be controlled based on the concentration of the solution.

For sufficient penetration of platinum or palladium into the zeolite catalytic material, the impregnation or immersion time of the solution is preferably 10 minutes or more and 5 hours or less.

The calcination temperature of the zeolite catalytic material is preferably 300°C or more and 600°C or less, and the calcination time of the zeolite catalytic material is preferably 30 minutes or more and 300 minutes or less.

The method for supporting phosphorus on the zeolite catalytic material is not particularly limited, but for example, an impregnation or immersion method can be used.

Orthophosphoric acid or phosphate ester can be used as a starting material for phosphorus when supporting phosphorus on the zeolite catalytic material. For example, an aqueous solution of orthophosphoric acid or phosphate ester can be used as an impregnation or immersion liquid.

Regarding the impregnation or immersion method when supporting phosphorus, after impregnating the zeolite catalytic material with a solution of orthophosphoric acid or phosphate ester (hereinafter referred to as "phosphoric acid solution") or after immersing the zeolite catalytic material in a phosphoric acid solution, and then calcining the impregnated or immersed zeolite catalytic material, P can be efficiently incorporated into the zeolite catalytic material, and the amount of P contained in the zeolite catalytic material can be easily controlled.

The concentration of the phosphoric acid solution is preferably 2 mass% or more and 20 mass% or less.

For sufficient penetration of the phosphoric acid solution into the zeolite catalytic material, the impregnation or immersion time of the phosphoric acid solution is preferably 10 minutes or more and 5 hours or less.

The calcination temperature of the zeolite catalytic material is preferably 300°C or more and 600°C or less. The calcination time of the zeolite catalytic material is preferably 30 minutes or more and 300 minutes or less.

The concentration of the phosphoric acid solution and the impregnation or immersion time of the phosphoric acid solution can control the content ratio of P.

After incorporating P into the zeolite catalytic material, it is preferable to support the noble metal such as platinum or palladium on the zeolite catalytic material. Specifically, for instance, after impregnating or immersing the zeolite catalytic material with the phosphoric acid solution, and then calcining the impregnated or immersed zeolite catalytic material, it is preferable to impregnate or immerse this calcined zeolite catalytic material in a solution containing noble metal such as platinum or palladium, and then calcine the zeolite catalytic material impregnated or immersed in the solution containing noble metal such as platinum or palladium.

The liquefied petroleum gas produced using the aforementioned catalyst for synthesizing liquefied petroleum gas contains a significant amount of propane as its component, and can therefore be stably used as an ideal fuel even in cold climates.

While embodiments have been described above, the present invention is not limited to these embodiments but encompasses all modifications within the scope of the present disclosure and the claims, and can be modified in various ways within the scope of the present disclosure.

### EXAMPLES

Next, Examples and Comparative Examples will be described, but the present disclosure is not limited to these Examples.

### <Example 1>

### (Cu-Zn-based Catalytic material)

A Cu-Zn-based catalytic material, specifically a ternary oxide of copper oxide, zinc oxide, and aluminum oxide (product name: 45776 Copper based methanol synthesis catalyst, manufactured by Alpha Aesar) was used. This Cu-Zn-based catalytic material was pelletized under a pressure of 5 MPa using a tablet press to produce pellets with a diameter of 20 mm and a thickness of about 1 mm; these pellets were then crushed in a mortar, and the crushed samples were sieved using overlaid 300 um and 500 um mesh sieves. As a result, a molded body composed of Cu-Zn-based catalytic material with a particle size of 300-500 um, granular shape, and a bulk density of 0.9 g/cm³ was obtained.

### (MFI-type Zeolite Catalytic Material)

10 g of MFI-type zeolite (ZSM-5, molar number of SiO₂ / molar number of Al₂O₃ = 40) was added to an agate mortar; an aqueous solution prepared by dissolving 0.1334 g of chloroplatinic acid hexahydrate in 7.5758 g of 10% hydrochloric acid was added dropwise using a pipette while mixing with a pestle to ensure uniformity; the impregnation took about 1 hour. Afterwards, the sample was dried at 100°C for 10 hours, and then calcined under an air atmosphere by raising the temperature from room temperature to 500°C for 50 minutes and maintaining this temperature for 120 minutes, resulting in an MFI-type zeolite supporting Pt. This sample was subsequently pelletized using a tablet press to produce pellets with a diameter of 20 mm and a thickness of about 1 mm, the pellets were then crushed in a mortar, and the crushed samples were sieved using overlaid 300 um and 500 um mesh sieves. This resulted in obtaining a molded body composed of an MFI-type zeolite catalytic material supporting Pt, with a particle size of 300-500 um, granular shape, and a bulk density of 0.8 g/cm³.

### (Production of Liquefied Petroleum Gas)

For the catalyst for synthesizing liquefied petroleum gas, a mixture of the molded body made from the Cu-Zn-based catalytic material obtained as mentioned above and the molded body made from the MFI-type zeolite catalytic material obtained as mentioned above was used. The ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material is listed in Table 1. It should be noted that M2 represents the combined total of the mass (Pt, Pd) of the supported noble metals, the mass of the MFI-type zeolite catalytic material supporting the noble metals, and the mass of P when P is incorporated. Using the catalyst for synthesizing liquefied petroleum gas, liquefied petroleum gas was produced under the following conditions:

First, the catalyst for synthesizing liquefied petroleum gas was subjected to a reduction treatment with hydrogen. Then, carbon monoxide and hydrogen were supplied to the catalyst for synthesizing liquefied petroleum gas at a Gas Hourly Space Velocity (GHSV) of 2000/h. While supplying carbon monoxide and hydrogen to the catalyst for synthesizing liquefied petroleum gas, the temperature (synthesis temperature) was controlled at 320°C and the pressure at 5.0 MPa to produce liquefied petroleum gas from carbon monoxide and hydrogen.

For the reaction synthesizing liquefied petroleum gas from a mixed gas of carbon monoxide and hydrogen (CO:H₂ = 1:2 (molar ratio)), a fixed-bed high-pressure circulating reactor was used. The reactor employed was made of stainless steel (with an inner diameter of 6.2 mm and a total length of 60 cm). The catalyst was packed in the center of the reactor, sandwiched between layers of glass wool. The reactor was placed in an electric furnace, and the temperature in the electric furnace was measured by a thermocouple inserted into the center of the furnace and controlled by PID. The temperature of the catalyst was measured using a thermocouple inserted into the center of the catalyst layer inside the reactor. Note that the temperature of the catalyst is the synthesis temperature. The reduction treatment of the catalyst for synthesizing liquefied petroleum gas was carried out by supplying H₂ to the catalyst inside the reactor at a flow rate of 40 ml/min at 380°C for 2 hours before synthesizing.

### (Filling the Catalyst in the Reactor)

The state of the catalyst for synthesizing liquefied petroleum gas filled in the reactor may be a physical mixture of the Cu-Zn-based catalytic material and the MFI-type zeolite catalytic material in a certain ratio. Alternatively, only the MFI-type zeolite catalytic material may be filled at the reactor outlet side, and the physical mixed catalyst of the Cu-Zn-based catalytic material and the MFI-type zeolite catalytic material may be filled at the reactor inlet side. Alternatively, a physical mixed catalyst of the Cu-Zn-based catalytic material and the MFI-type zeolite catalytic material in a certain ratio may be filled at the reactor outlet side, a physical mixed catalyst of the Cu-Zn-based catalytic material and the MFI-type zeolite catalytic material in a certain ratio may be filled at the reactor inlet side. Alternatively, only the MFI-type zeolite catalytic material may be filled at the reactor outlet side; followed by filling the physically mixed catalyst of the Cu-Zn-based catalytic material and the MFI-type zeolite catalytic material in a certain ratio; next, only the MFI-type zeolite catalytic material may be filled; followed by filling the physically mixed catalyst of the Cu-Zn-based catalytic material and the MFI-type zeolite catalytic material in another ratio, in other words, the reactor may thus be filled with multiple layers of the MFI-type zeolite catalytic material, the Cu-Zn-based catalytic material and the MFI-type zeolite catalytic material. Alternatively, the physically mixed catalyst of the Cu-Zn-based catalytic material and the MFI-type zeolite catalytic material in a certain ratio may be filled on the reactor outlet side; subsequently, the physically mixed catalyst of the Cu-Zn-based catalytic material and the MFI-type zeolite catalytic material in another ratio may be filled; then, the physically mixed catalyst of the Cu-Zn-based catalytic material and the MFI-type zeolite catalytic material in a certain ratio may be filled; then, the physically mixed catalyst of the Cu-Zn-based catalytic material and the MFI-type zeolite catalytic material in another ratio may be filled, in other words, the reactor may thus be filled with multiple layers of the physically mixed catalyst of the Cu-Zn-based catalytic material and the MFI-type zeolite catalytic material in a certain ratio, and the physically mixed catalyst of the Cu-Zn-based catalytic material and the MFI-type zeolite catalytic material in another ratio.

### (Gas Composition Analysis)

At a predetermined point of time after starting the reaction (6 hours later), the gas was analyzed using a gas chromatograph connected online. The gas chromatograph used was GC-2014 (manufactured by Shimadzu Corporation). The analysis target and the conditions are described below.

### <Analysis Conditions>

Column: RT-Q-BOND
Temperature program:
   (i) 45°C (held for 30 min)
   (ii) Heated at 2°C/min to 175°C
   (iii) 175°C (held for 40 min)
Analysis time: 135 min.

### [Measurement and Evaluation]

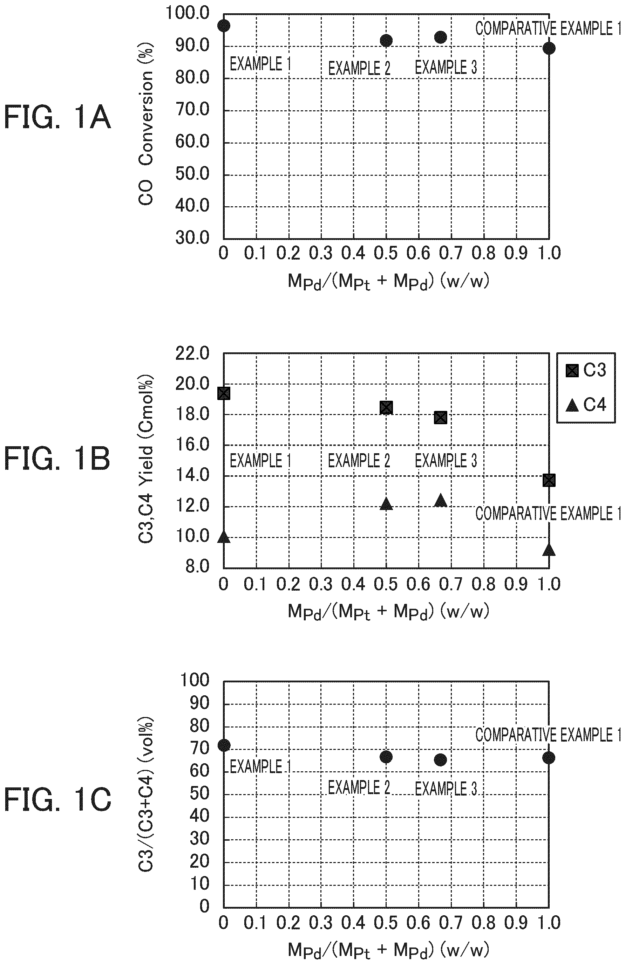

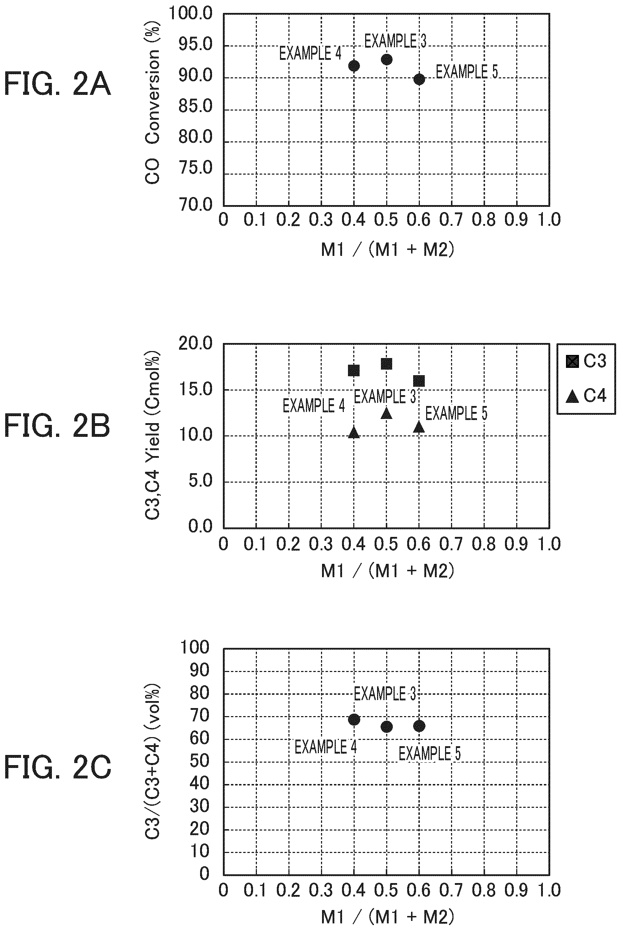

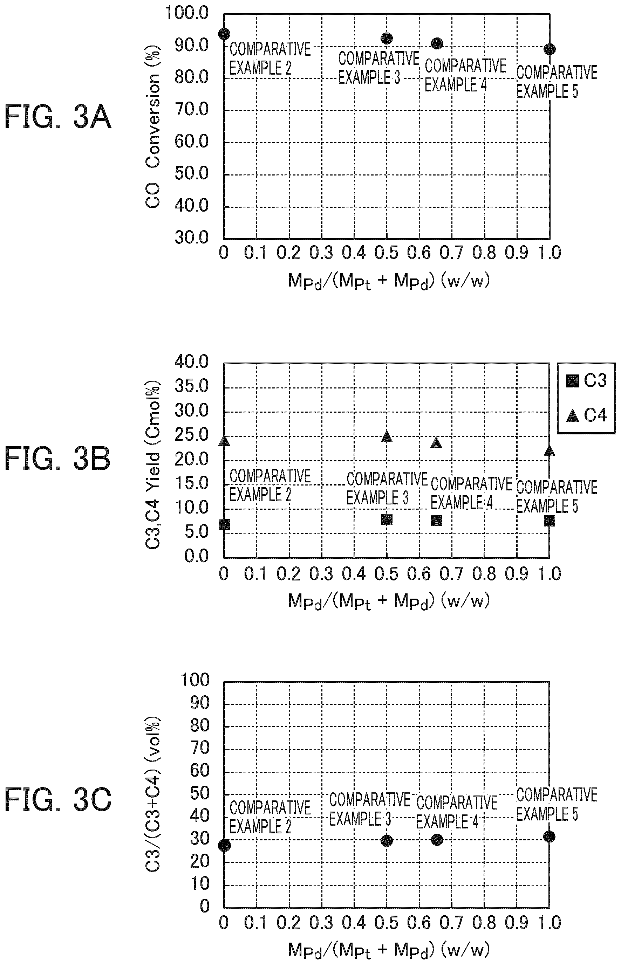

For the catalyst for synthesizing liquefied petroleum gas used in the Examples and Comparative Examples, and for the liquefied petroleum gas obtained in the Examples and Comparative Examples, the following measurements and evaluations were performed. The results are illustrated in Tables 1 to 4 and Figs. 1 to 3. In the drawings, the results of Examples 1 to 3 and Comparative Example 1 are illustrated in Fig. 1, the results of Examples 3 to 5 are illustrated in Fig. 2, and the results of Comparative Examples 2 to 5 are illustrated in Fig. 3. The results for the liquefied petroleum gas were measured 6 hours after the start of the reaction with carbon monoxide and hydrogen.
[1] Molar Ratio (Molar Number of SiO₂ / Molar Number of Al₂O₃)
   The SiO₂/Al₂O₃ ratio (molar number of SiO₂ / molar number of Al₂O₃) was measured by ICP-OES (Inductively Coupled Plasma Optical Emission Spectroscopy).
[2] Presence or absence of Pd and Pt, Ratio (((M_{Pt}+M_{Pd})/M2) × 100) of Total Mass (M_{Pt}+M_{Pd}) of Mass (M_{Pt}) of Pt and Mass (M_{pd}) of Pd to Mass (M2) of MFI-type Zeolite Catalytic Material, and Ratio (M_{Pd}/(M_{Pt}+M_{Pd})) of Mass (M_{pd}) of Pd to Total Mass (M_{Pt}+M_{Pd}) of Mass (M_{Pt}) of Pt and Mass (M_{Pd}) of Pd
   The presence or absence of Pd and Pt supported on the MFI-type zeolite catalytic material, the ratio (((M_{Pt}+M_{Pd})/M2) × 100), and the ratio (M_{Pd}/(M_{Pt}+M_{Pd})) were measured by ICP-OES (Inductively Coupled Plasma Optical Emission Spectroscopy).
[3] Presence or absence of P, and Content Ratio of P (Ratio ((M_{P}/M2) × 100)) of Mass (M_{P}) of P in MFI-type Zeolite Catalytic Material to Mass (M2) of MFI-type Zeolite Catalytic Material)
   The presence or absence of P in the MFI-type zeolite catalytic material and the content ratio of P in the MFI-type zeolite catalytic material were measured by ICP-OES (Inductively Coupled Plasma Optical Emission Spectroscopy).
[4] CO Conversion Rate (CO Conversion as illustrated in the drawings) CO Conversation Rate (%) = [(CO flow rate at the inlet (µmol/min) - CO flow rate at the outlet (µmol/min))/ CO flow rate at the inlet (µmol/min)] × 100 The CO conversion rate indicates the ratio of carbon monoxide (CO) in the reaction raw gas being converted to hydrocarbons and the like.
[5] Propane Yield (C3 Yield as illustrated in the drawings) Propane Yield (Cmol%) = [(C3 Production Rate × 3) / (Inlet CO Flow Rate) × 106/22400] × 100 The unit of the C3 production rate is Cumol/min, and the unit of the inlet CO flow rate is ml (Normal)/min. C3 represents propane.
[6] Butane Yield (C4 Yield as illustrated in the drawings) Butane Yield (Cmol%) = [(C4 Production Rate × 4) / (Inlet CO Flow Rate) × 106/22400] × 100 The unit of the C4 production rate is Cumol/min, and the unit of the inlet CO flow rate is ml (Normal)/min. C4 represents butane.
[7] Ratio of Propane to Combined Total of Propane and Butane (C3/(C3+C4) Ratio as illustrated in the drawings) Ratio of propane to the combined total of propane and butane = [molar number of propane / (molar number of propane + molar number of butane)] × 100
[8] Initial Catalyst Activity (Initial Performance) of Catalyst for Synthesizing Liquefied Petroleum Gas

Ranking was done for the initial performance of the catalyst for synthesizing liquefied petroleum gas. Specifically, the LPG yield in the initial performance and the propane yield in the initial performance were ranked based on the criteria below, and the lower rank between the LPG yield in the initial performance and the propane yield in the initial performance was considered as the overall evaluation of the initial performance. Ranks A, B, and C for the overall evaluation of the initial performance are considered as "pass", while rank D is considered "fail".

### <LPG Yield in Initial Performance (Liquefied Petroleum Gas (Propane + Butane) Yield Cmol%)>

A: The yield of liquefied petroleum gas (propane + butane) is 30.0 Cmol% or more, 6 hours after the start of liquefied petroleum gas production.
B: The yield of liquefied petroleum gas (propane + butane) is 25.0 Cmol% or more and less than 30.0 Cmol%, 6 hours after the start of liquefied petroleum gas production.
C: The yield of liquefied petroleum gas (propane + butane) is 20.0 Cmol% or more and less than 25.0 Cmol%, 6 hours after the start of liquefied petroleum gas production.
D: The yield of liquefied petroleum gas (propane + butane) is less than 20.0 Cmol%, 6 hours after the start of liquefied petroleum gas production.

### <Propane Yield in Initial Performance (Propane Yield Cmol%)>

A: Propane yield is 20.0 Cmol% or more, 6 hours after the start of liquefied petroleum gas production.
B: Propane yield is 15.0 Cmol% or more and less than 20.0 Cmol%, 6 hours after the start of liquefied petroleum gas production.
C: Propane yield is 10.0 Cmol% or more and less than 15.0 Cmol%, 6 hours after the start of liquefied petroleum gas production.
D: Propane yield is less than 10.0 Cmol%, 6 hours after the start of liquefied petroleum gas production.

### [9] Catalyst Activity after One Week (Mid-Term Performance) of Catalyst for Synthesizing Liquefied Petroleum Gas

Ranking was done for the mid-term performance of the catalyst for synthesizing liquefied petroleum gas. Ranks A, B, and C are considered as "pass", while rank D is considered "fail".
A: The ratio of the yield of liquefied petroleum gas one week after the start of liquefied petroleum gas production to the yield (Cmol%) of the liquefied petroleum gas (propane + butane) 6 hours after the start of liquefied petroleum gas production is 90.0% or more.
B: The ratio of the yield of liquefied petroleum gas one week after the start of liquefied petroleum gas production to the yield (Cmol%) of the liquefied petroleum gas (propane + butane) 6 hours after the start of liquefied petroleum gas production is 80.0% or more and less than 90.0%.
C: The ratio of the yield of liquefied petroleum gas one week after the start of liquefied petroleum gas production to the yield (Cmol%) of the liquefied petroleum gas (propane + butane) 6 hours after the start of liquefied petroleum gas production is 60.0% or more and less than 80.0%.
D: The ratio of the yield of liquefied petroleum gas one week after the start of liquefied petroleum gas production to the yield (Cmol%) of the liquefied petroleum gas (propane + butane) 6 hours after the start of liquefied petroleum gas production is less than 60.0%.

### [10] Catalyst Activity after One Month (Long-Term Performance) of Catalyst for Synthesizing Liquefied Petroleum Gas

Ranking was done for the long-term performance of the catalyst for synthesizing liquefied petroleum gas. Ranks A, B, and C are considered as "pass", while rank D is considered "fail".
A: The ratio of the yield of liquefied petroleum gas one month after the start of liquefied petroleum gas production to the yield (Cmol%) of the liquefied petroleum gas 6 hours after the start of liquefied petroleum gas production is 90.0% or more.
B: The ratio of the yield of liquefied petroleum gas one month after the start of liquefied petroleum gas production to the yield (Cmol%) of the liquefied petroleum gas 6 hours after the start of liquefied petroleum gas production is 80.0% or more and less than 90.0%.
C: The ratio of the yield of liquefied petroleum gas one month after the start of liquefied petroleum gas production to the yield (Cmol%) of the liquefied petroleum gas 6 hours after the start of liquefied petroleum gas production is 60.0% or more and less than 80.0%.
D: The ratio of the yield of liquefied petroleum gas one month after the start of liquefied petroleum gas production to the yield (Cmol%) of the liquefied petroleum gas 6 hours after the start of liquefied petroleum gas production is less than 60.0%.

### <Example 2>

The operations were carried out in the same manner as in Example 1, except that an aqueous solution prepared by dissolving 0.0667 g of chloroplatinic acid hexahydrate and 0.0419 g of palladium chloride in 7.5758 g of 10% hydrochloric acid was used instead of the aqueous solution prepared by dissolving 0.1334 g of chloroplatinic acid hexahydrate in 7.5758 g of 10% hydrochloric acid.

### <Example 3>

The operations were carried out in the same manner as in Example 1, except that an aqueous solution prepared by dissolving 0.0419 g of chloroplatinic acid hexahydrate and 0.0574 g of palladium chloride in 7.5758 g of 10% hydrochloric acid was used instead of the aqueous solution prepared by dissolving 0.1334 g of chloroplatinic acid hexahydrate in 7.5758 g of 10% hydrochloric acid.

### <Comparative Example 1>

The operations were carried out in the same manner as in Example 1, except that an aqueous solution prepared by dissolving 0.0837 g of palladium chloride in 7.5758 g of 10% hydrochloric acid was used instead of the aqueous solution prepared by dissolving 0.1334 g of chloroplatinic acid hexahydrate in 7.5758 g of 10% hydrochloric acid.

### <Examples 4 and 5>

The operations were carried out in the same manner as in Example 3, except that the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material was matched to the value listed in Table 1.

### <Comparative Examples 2 to 5>

The operations were carried out in the same manner as in Examples 1 to 3 and Comparative Example 1, except that β-type zeolite (molar number of SiO₂/ molar number of Al₂O₃ = 40) was used instead of the MFI-type zeolite.

**[Table 1]**

| | M1/ (M1 + M2) | Zeolite catalytic material | | | | | Reaction temperature (°C) | Pressure (MPa) | GHSV (h⁻¹) | Initial performance (6 hours after the start of operation) | | | | | Overall evaluation of initial performance | LPG yield in initial performance | Propane yield in initial performance |
|---|---|---|---|---|---|---|---|---|---|---|---|---|---|---|---|---|---|
| | | Supported element | M_{Pd}/ (M_{Pd}+M_{Pt}) | ((M_{Pd}+M_{Pt})/M2) ×100 (mass %) | (M_{P}/M2) ×100 (mass %) | Zeolite | | | | CO conversion rate (%) | LPG yield (C-mol%) | Propane yield (C-mol%) | Butane yield (C-mol%) | Propane/ (propane + butane) (Vol%) | | | |
| Example 1 | 0.50 | Pt | 0.00 | 0.50 | None | M F I | 320 | 5.0 | 2000 | 96.5 | 29.5 | 19.4 | 10.1 | 72.0 | B | B | B |
| Example 2 | 0.50 | Pt, Pd | 0.50 | 0.50 | None | M F I | 320 | 5.0 | 2000 | 91.9 | 30.7 | 18.5 | 12.2 | 66.9 | B | A | B |
| Example 3 | 0.50 | Pt, Pd | 0.67 | 0.50 | None | M F I | 320 | 5.0 | 2000 | 92.9 | 30.3 | 17.8 | 12.5 | 65.6 | B | A | B |
| Example 4 | 0.40 | Pt, Pd | 0.67 | 0.50 | None | M F I | 320 | 5.0 | 2000 | 91.9 | 27.5 | 17.1 | 10.4 | 68.7 | B | B | B |
| Example 5 | 0.60 | Pt, Pd | 0.67 | 0.50 | None | M F I | 320 | 5.0 | 2000 | 89.8 | 27.0 | 16.0 | 11.0 | 66.0 | B | B | B |
| Example 6 | 0.50 | Pt, Pd | 0.67 | 0.50 | 2.0 | M F I | 320 | 5.0 | 2000 | 92.9 | 28.1 | 21.2 | 6.8 | 80.6 | B | B | A |
| Comparative Example 1 | 0.50 | Pd | 1.00 | 0.50 | None | M F I | 320 | 5.0 | 2000 | 89.4 | 23.0 | 13.7 | 9.2 | 66.5 | C | C | C |
| Comparative Example 2 | 0.50 | Pt | 0.00 | 0.50 | None | Beta-type | 320 | 5.0 | 2000 | 93.9 | 31.1 | 6.9 | 24.2 | 27.5 | D | A | D |
| Comparative Example 3 | 0.50 | Pt, Pd | 0.50 | 0.50 | None | Beta-type | 320 | 5.0 | 2000 | 92.5 | 32.9 | 7.9 | 25.0 | 29.6 | D | A | D |
| Comparative Example 4 | 0.50 | Pt, Pd | 0.67 | 0.50 | None | Beta-type | 320 | 5.0 | 2000 | 91.0 | 31.4 | 7.7 | 23.8 | 30.1 | D | A | D |
| Comparative Example 5 | 0.50 | Pd | 1.00 | 0.50 | None | Beta-type | 320 | 5.0 | 2000 | 89.1 | 29.7 | 7.6 | 22.1 | 31.5 | D | B | D |
| Comparative Example 6 | 0.20 | Pt, Pd | 0.68 | 0.50 | 2.0 | M F I | 320 | 5.0 | 2000 | 77.7 | 18.4 | 11.9 | 6.5 | 70.9 | D | D | C |

As illustrated in Table 1 and Figs. 1 to 3, Examples 1 to 6, which included the Cu-Zn-based catalytic material and the MFI-type zeolite catalytic material supporting Pt, with the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material being within the range of 0.30 or more and 0.95 or less, had high propane yield even at a low synthesis temperature of 320°C. Moreover, the ratio of propane to the combined total of propane and butane was higher in Examples 1 to 6 compared to Comparative Examples 2 to 5 which used β-zeolite. Additionally, for the mass ratio M_{Pd}/(M_{Pt}+M_{Pd}) illustrated in Fig. 1, Examples 2 and 3, which were within the range of 0.15 or more and 0.70 or less, had higher butane yield compared to Example 1, which was outside of this range. On the other hand, Comparative Example 1, which supported Pd without Pt, and Comparative Examples 2 to 5, which used β-zeolite, had lower propane yields. Note that the CO conversion rate was over 80% in both the Examples and Comparative Examples, indicating that CO was adequately reacted.

### <Example 6>

The operations were carried out in the same manner as in Example 3, except for the following: 10 g of MFI-type zeolite (ZSM-5, molar number of SiO₂ / molar number of Al₂O₃ = 40) was added to an agate mortar; an aqueous solution prepared by dissolving 0.6456 g of orthophosphoric acid in 6.000 g of deionized water was added dropwise using a pipette while mixing with a pestle to ensure uniformity; the impregnation took about 1 hour; the sample was dried at 100°C for 10 hours, then calcined under an air atmosphere by raising the temperature from room temperature to 500°C for 50 minutes and maintaining this temperature for 120 minutes; and an aqueous solution prepared by dissolving 0.0419 g of chloroplatinic acid hexahydrate and 0.0574 g of palladium chloride in 7.5758 g of 10% hydrochloric acid was added dropwise to the calcined sample using a pipette.

### <Example 7>

### (Cu-Zn-based Catalytic material)

95.13 g of copper nitrate trihydrate, 49.73 g of zinc nitrate hexahydrate, 19.33 g of aluminum nitrate nonahydrate, and 5.31 g of zirconyl nitrate dihydrate were dissolved in 584 g of distilled water to prepare Solution A. Additionally, 148 g of anhydrous sodium carbonate was dissolved in 2000 g of distilled water to prepare Solution B.

A 5000 ml separable flask with a stirrer was charged with 500 g of distilled water and heated in a water bath until the water reached 65°C. The entirety of Solution A and 900 ml of Solution B were added to the separable flask by setting the rate of a liquid delivery pump to complete the addition in 70 minutes. Vigorous stirring was maintained during the addition. The precipitate slurry temperature was adjusted to 65°C with the water bath, as needed. After the addition of Solution A and Solution B, the pH was approximately 5.6. The remaining portion of Solution B was then slowly added until the pH reached 6.5. After reaching pH 6.5, the precipitate slurry was stirred vigorously at 65°C for 2 hours. The pH after 2 hours was approximately 7.2.

Subsequently, the precipitation slurry was transferred to a suction filtration apparatus and filtered to obtain a precipitate cake. The obtained precipitate cake was washed 20 times with 250 ml of distilled water to remove Na ion from the precipitate cake.

After washing, the precipitate cake was transferred to an evaporating dish and dried in a drying oven at 120°C for 12 hours. It was then heated in a calcination furnace at a rate of 10°C/min up to 350°C and calcined at 350°C for 2 hours; the resulting calcined product was then thoroughly ground in an agate mortar to produce a powder. This powder was pelletized under a pressure of 5 MPa using a tablet press to produce pellets with a diameter of 20 mm and a thickness of about 1 mm, the pellets were then crushed in a mortar, and the crushed samples were sieved using overlaid 300 um and 500 um mesh sieves. As a result, a molded body composed of Cu-Zn-based catalytic material with a particle size of 300-500 um, granular shape, and a bulk density of 0.9 g/cm³ was obtained.

The Cu-Zn-based catalytic material consisted of copper oxide (CuO), zinc oxide (ZnO), zirconium oxide (ZrO₂), and aluminum oxide (Al₂O₃), and had a chemical composition with 62.7 mass% copper oxide, 27.3 mass% zinc oxide, 5.0 mass% zirconium oxide, and 5.3 mass% aluminum oxide.

### (MFI-type Zeolite Catalytic Material)

36 g of MFI-type zeolite (ZSM-5, molar number of SiO₂ / molar number of Al₂O₃ = 40) was added to an agate mortar; an aqueous solution prepared by dissolving 2.3241 g of orthophosphoric acid in 21.6 g of deionized water was added dropwise using a pipette while mixing with a pestle to ensure uniformity; the impregnation took about 1 hour. Afterwards, the sample was dried at 100°C for 10 hours and then calcined under an air atmosphere by raising the temperature from room temperature to 500°C for 50 minutes and maintaining this temperature for 120 minutes.

30 g of the calcined MFI-type zeolite containing P was added to an agate mortar; and an aqueous solution prepared by dissolving 0.4002 g of chloroplatinic acid hexahydrate in 22.7274 g of 10% hydrochloric acid was added dropwise using a pipette while mixing with a pestle to ensure uniformity; the impregnation took about 1 hour. Afterwards, the sample was dried at 100°C for 10 hours, and then calcined under an air atmosphere by raising the temperature from room temperature to 500°C for 50 minutes and maintaining this temperature for 120 minutes, resulting in an MFI-type zeolite containing P and supporting Pt. The sample was pelletized using a tablet press to produce pellets with a diameter of 20 mm and a thickness of about 1 mm; the pellets were then crushed in a mortar, and the crushed samples were sieved using overlaid 300 um and 500 um mesh sieves. As a result, a molded body composed of the MFI-type zeolite catalytic material containing P and supporting Pt, with a particle size of 300-500 um, granular shape, and a bulk density of 0.8 g/cm³ was obtained.

The chemical composition of this MFI-type zeolite catalytic material was 0.5 mass% platinum ((M_{Pt}/M2) × 100 being 0.50 mass%) and 2.0 mass% phosphorus ((M_{P}/M2) × 100 being 2.0 mass%), with the remainder being ZSM-5.

### (Production of Liquefied Petroleum Gas)

For the catalyst for synthesizing liquefied petroleum gas, a mixture of the molded body made from the Cu-Zn-based catalytic material obtained as mentioned above and the molded body made from the MFI-type zeolite catalytic material obtained as mentioned above was used. The mixture was prepared such that the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material was 0.80. M2 represents the combined total of the mass of the supported noble metal (Pt), the mass of the MFI-type zeolite catalytic material supporting the noble metal (Pt), and the mass of P. Using the catalyst for synthesizing liquefied petroleum gas, liquefied petroleum gas was produced under the following conditions.

First, the catalyst for synthesizing liquefied petroleum gas was subjected to a reduction treatment with hydrogen. Then, carbon monoxide and hydrogen were supplied to the catalyst for synthesizing liquefied petroleum gas at a gas hourly space velocity (GHSV) of 750/h. While supplying carbon monoxide and hydrogen to the catalyst for synthesizing liquefied petroleum gas, the temperature (synthesis temperature) was controlled at 320°C and the pressure at 5.0 MPa to produce liquefied petroleum gas from carbon monoxide and hydrogen.

For the reaction synthesizing liquefied petroleum gas from a mixed gas of carbon monoxide and hydrogen (CO:H₂ = 1:2 (molar ratio)), a fixed-bed high-pressure circulating reactor was used. The reactor employed was made of stainless steel (with an inner diameter of 6.2 mm and a total length of 60 cm). The catalyst was packed in the center of the reactor, sandwiched between layers of glass wool. The reactor was placed in an electric furnace, and the temperature in the electric furnace was measured by a thermocouple inserted into the center of the furnace and controlled by PID. The temperature of the catalyst was measured using a thermocouple inserted into the center of the catalyst layer inside the reactor. Note that the temperature of the catalyst is the synthesis temperature. The reduction treatment of the catalyst for synthesizing liquefied petroleum gas was carried out by supplying H₂ to the catalyst inside the reactor at a flow rate of 40 ml/min at 380°C for 2 hours before synthesizing.

### (Gas Composition Analysis)

At predetermined points of time after starting the reaction (6 hours later, 1 week later, 1 month later), the gas was analyzed using a gas chromatograph connected online. The gas chromatograph used was GC-2014 (manufactured by Shimadzu Corporation). The analysis target and the conditions are described below.

### <Analysis Conditions>

Column: RT-Q-BOND
Temperature Program:
   (i) 45°C (held for 30 min)
   (ii) Heated at 2°C/min to 175°C
   (iii) 175°C (held for 40 min)
Analysis Time: 135 min

### [Measurement and Evaluation]

Performance evaluations were carried out in a manner similar to the above for the catalyst for synthesizing liquefied petroleum gas, as well as for the liquefied petroleum gas obtained in the aforementioned Examples and Comparative Examples.

### <Example 8>

The molded body composed of the Cu-Zn-based catalytic material and the molded body composed of the MFI-type zeolite catalytic material identical to those in Example 7 were mixed to prepare a catalyst for use, and the same operations as in Example 7 were performed, except that the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material was set to 0.78, and the temperature (synthesis temperature) was set to 310°C.

### <Example 9>

The molded body composed of the Cu-Zn-based catalytic material and the molded body composed of the MFI-type zeolite catalytic material identical to those in Example 7 were mixed to prepare a catalyst (mixed catalyst), and the same operations as in Example 7 were performed, except that the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material was set to 0.72 to obtain the catalyst, the catalyst (mixed catalyst) and a catalyst solely composed of MFI-type zeolite catalytic material was used, the catalyst solely composed of MFI-type zeolite catalytic material was filled in the lower section of the reactor, and silica wool was inserted before filling the upper section with the mixed catalyst, and the temperature (synthesis temperature) was set to 300°C.

### <Example 10>

The molded body composed of the Cu-Zn-based catalytic material and the molded body composed of the MFI-type zeolite catalytic material identical to those in Example 7 were mixed to prepare a catalyst (mixed catalyst), and the same operations as in Example 7 were performed, except that the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material was set to 0.64 to obtain the catalyst, the catalyst (mixed catalyst) and a catalyst solely composed of MFI-type zeolite catalytic material was used, the catalyst solely composed of MFI-type zeolite catalytic material was filled in the lower section of the reactor, and silica wool was inserted before filling the upper section with the mixed catalyst, and the temperature (synthesis temperature) was set to 300°C.

### <Example 11>

The molded body composed of the Cu-Zn-based catalytic material and the molded body composed of the MFI-type zeolite catalytic material identical to those in Example 7 were mixed to prepare a catalyst for use, and the same operations as in Example 7 were performed, except that the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material was set to 0.50, the temperature (synthesis temperature) was set to 280°C, and the GHSV was set to 1000 h-1

### <Example 12>

### (Cu-Zn-based Catalytic material)

The Cu-Zn-based catalytic material used was a ternary oxide composed of copper oxide, zinc oxide, and aluminum oxide (Product Name: 45776 Copper based methanol synthesis catalyst, manufactured by Alpha Aesar). This Cu-Zn-based catalytic material was pelletized under a pressure of 5 MPa using a tablet press to produce pellets with a diameter of 20 mm and a thickness of about 1 mm; these pellets were then crushed in a mortar, and the crushed samples were sieved using overlaid 300 um and 500 um mesh sieves. As a result, a molded body composed of Cu-Zn-based catalytic material with a particle size of 300-500 um, granular shape, and a bulk density of 0.9 g/cm³ was obtained.

This Cu-Zn-based catalytic material was composed of copper oxide (CuO), zinc oxide (ZnO), magnesium oxide (MgO), and aluminum oxide (Al₂O₃); and the chemical composition was 63.5 mass% copper oxide, 25.0 mass% zinc oxide, 1.5 mass% magnesium oxide, and 10.0 mass% aluminum oxide.

### (MFI-type Zeolite Catalytic Material)

12 g of MFI-type zeolite (ZSM-5, molar number of SiO₂ / molar number of Al₂O₃ = 40) was added to an agate mortar; an aqueous solution prepared by dissolving 0.3834 g of orthophosphoric acid in 7.2000 g of deionized water was added dropwise using a pipette while mixing with a pestle to ensure uniformity; the impregnation took about 1 hour; the sample was dried at 100°C for 10 hours, and then calcined under an air atmosphere by raising the temperature from room temperature to 500°C for 50 minutes and maintaining this temperature for 120 minutes.

10 g of the calcined MFI-type zeolite containing P was added to an agate mortar; and an aqueous solution prepared by dissolving 0.0419 g of chloroplatinic acid hexahydrate and 0.0574 g of palladium chloride in 7.5758 g of 10% hydrochloric acid was added dropwise using a pipette while mixing with a pestle to ensure uniformity; the impregnation took about 1 hour. Afterwards, the sample was dried at 100°C for 10 hours, and then calcined under an air atmosphere by raising the temperature from room temperature to 500°C for 50 minutes and maintaining this temperature for 120 minutes, resulting in an MFI-type zeolite containing P and supporting both Pt and Pd. The sample was pelletized using a tablet press to produce pellets with a diameter of 20 mm and a thickness of about 1 mm; the pellets were then crushed in a mortar, and the crushed samples were sieved using overlaid 300 um and 500 um mesh sieves. As a result, a molded body composed of the MFI-type zeolite catalytic material containing P and supporting both Pt and Pd, with a particle size of 300-500um, granular shape, and a bulk density of 0.8 g/cm³ was obtained.

The chemical composition of this MFI-type zeolite catalytic material was 0.16 mass% platinum + 0.34 mass% palladium ((M_{Pt}+M_{Pd})/M2) × 100 being 0.50 mass%), 2.0 mass% phosphorus ((M_{P}/M2) × 100 being 2.0 mass%), with the remainder being ZSM-5.

### (Production of Liquefied Petroleum Gas)

For the catalyst for synthesizing liquefied petroleum gas, a mixture of the molded body made from the Cu-Zn-based catalytic material obtained as mentioned above and the molded body made from the MFI-type zeolite catalytic material obtained as mentioned above was used. Using a catalyst, in which the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material was 0.50, the temperature (synthesis temperature) was set to 320°C, and the GHSV was set to 2000 h⁻¹. Other operations were carried out in the same manner as in Example 7. The evaluation was conducted for 25 days.

### <Comparative Example 6>

The molded body composed of the Cu-Zn-based catalytic material and the molded body composed of the MFI-type zeolite catalytic material identical to those in Example 12 were mixed to prepare a catalyst for use, and the same operations as in Example 12 were performed, except that the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material was set to 0.20, and the temperature (synthesis temperature) was set to 320°C.

### [Table 2]

| | M1/ (M1 + M2) | Zeolite catalytic material | | | | | Reaction temperature (t) | Pressure (MPa) | GHSV (h⁻¹) | Initial performance (6 hours after the start of operation) | | | | | Overall evaluation of initial performance | LPG yield in initial performance | Propane yield in initial performance |
|---|---|---|---|---|---|---|---|---|---|---|---|---|---|---|---|---|---|
| | | Supported element | M_{Pd}/ (M_{Pd}+M_{Pt}) | ((M_{Pd}+M_{Pt})/M2) × 100 (mass %) | (M _{P}/M2) × 100 (mass %) | Zeolite | | | | CO conversion rate (%) | LPG yield (C-mol%) | Propane yield (C-mol%) | Butane yield (C-mol%) | Propane/ (propane + butane) (Vol%) | | | |
| Example 7 | 0.80 | Pt | 0.00 | 0.50 | 2.0 | M F I | 320 | 5.0 | 750 | 92.6 | 32.3 | 25.0 | 7.4 | 81.9 | A | A | A |
| Example 8 | 0.78 | Pt | 0.00 | 0.50 | 2.0 | M F I | 310 | 5.0 | 750 | 91.8 | 30.0 | 23.6 | 6.4 | 83.2 | A | A | A |
| Example 9 | 0.72 | Pt | 0.00 | 0.50 | 2.0 | M F I | 300 | 5.0 | 750 | 91.0 | 31.8 | 23.7 | 8.1 | 79.5 | A | A | A |
| Example 10 | 0.64 | Pt | 0.00 | 0.50 | 2.0 | M F I | 300 | 5.0 | 750 | 93.5 | 30.7 | 23.7 | 7.0 | 81.9 | A | A | A |
| Example 11 | 0.50 | Pt | 0.00 | 0.50 | 2.0 | M F I | 280 | 5.0 | 1000 | 83.9 | 27.1 | 21.6 | 5.5 | 83.9 | B | B | A |
| Example 12 | 0.50 | Pt, Pd | 0.68 | 0.50 | 2.0 | M F I | 320 | 5.0 | 2000 | 92.9 | 30.3 | 17.8 | 12.5 | 65.6 | B | A | B |

**[Table 3]**

| | M 1/ (M1 + M2) | Zeolite catalytic material | | | | | Reaction temperature (°C) | Pressure (MPa) | GHSV (h⁻¹) | Mid-term performance (1 week after the start of operation) | | | | | Evaluation of mid-term performance |
|---|---|---|---|---|---|---|---|---|---|---|---|---|---|---|---|
| | | Supported element | M_{Pd}/ (M_{Pd}+M_{Pt}) | ((M_{Pd}+M_{Pt})/M2) ×100 (mass %) | (M_{P}/M2) ×100 (mass %) | Zeolite | | | | CO conversion rate (%) | LPG yield (C-mol%) | Propane yield (C-mol%) | Butane yield (C-mol%) | Propane/ (propane + butane) (Vol%) | |
| Example 7 | 0.80 | Pt | 0.00 | 0.50 | 2.0 | M F I | 320 | 5.0 | 750 | 92.9 | 32.6 | 24.8 | 7.8 | 80.9 | A |
| Example 8 | 0.78 | Pt | 0.00 | 0.50 | 2.0 | M F I | 310 | 5.0 | 750 | 94.0 | 33.1 | 25.6 | 7.5 | 82.0 | A |
| Example 9 | 0.72 | Pt | 0.00 | 0.50 | 2.0 | M F I | 300 | 5.0 | 750 | 89.6 | 29.3 | 21.7 | 7.6 | 79.1 | A |
| Example 10 | 0.64 | Pt | 0.00 | 0.50 | 2.0 | M F I | 300 | 5.0 | 750 | 84.1 | 27.5 | 20.4 | 7.1 | 79.2 | B |
| Example 11 | 0.50 | Pt | 0.00 | 0.50 | 2.0 | M F I | 280 | 5.0 | 1000 | 55.3 | 17.0 | 12.9 | 4.1 | 81.1 | C |
| Example 12 | 0.50 | Pt, Pd | 0.68 | 0.50 | 2.0 | M F I | 320 | 5.0 | 2000 | 76.5 | 24.9 | 13.8 | 11.1 | 62.4 | B |

**[Table 4]**

| | M1/ (M1 + M2) | Zeolite catalytic material | | | | | Reaction temperature (°C) | Pressure (MPa) | GHSV (h⁻¹) | Long-term performance (1 month after the start of operation) | | | | | Evaluation of long-term performance |
|---|---|---|---|---|---|---|---|---|---|---|---|---|---|---|---|
| | | Supported element | M_{Pd}/ (M_{Pd}+M_{Pt}) | ((M_{Pd}+M_{Pt})/M2) × 100 (mass %) | (M _{P}/M2) × 100 (mass %) | Zeolite | | | | CO conversion rate (%) | LPG yield (C-mol%) | Propane yield (C-mol%) | Butane yield (C-mol%) | Propane/ (propane + butane) (Vol%) | |
| Example 7 | 0.80 | Pt | 0.00 | 0.50 | 2.0 | M F I | 320 | 5.0 | 750 | 91.0 | 30.0 | 22.3 | 7.8 | 79.3 | A |
| Example 8 | 0.78 | Pt | 0.00 | 0.50 | 2.0 | M F I | 310 | 5.0 | 750 | 88.3 | 29.8 | 22.7 | 7.2 | 80.8 | A |
| Example 9 | 0.72 | Pt | 0.00 | 0.50 | 2.0 | M F I | 300 | 5.0 | 750 | 81.3 | 25.7 | 18.4 | 7.3 | 77.2 | B |
| Example 10 | 0.64 | Pt | 0.00 | 0.50 | 2.0 | M F I | 300 | 5.0 | 750 | - | - | - | - | - | - |
| Example 11 | 0.50 | Pt | 0.00 | 0.50 | 2.0 | M F I | 280 | 5.0 | 1000 | - | - | - | - | - | - |
| Example 12 | 0.50 | Pt, Pd | 0.68 | 0.50 | 2.0 | M F I | 320 | 5.0 | 2000 | - | - | - | - | - | - |

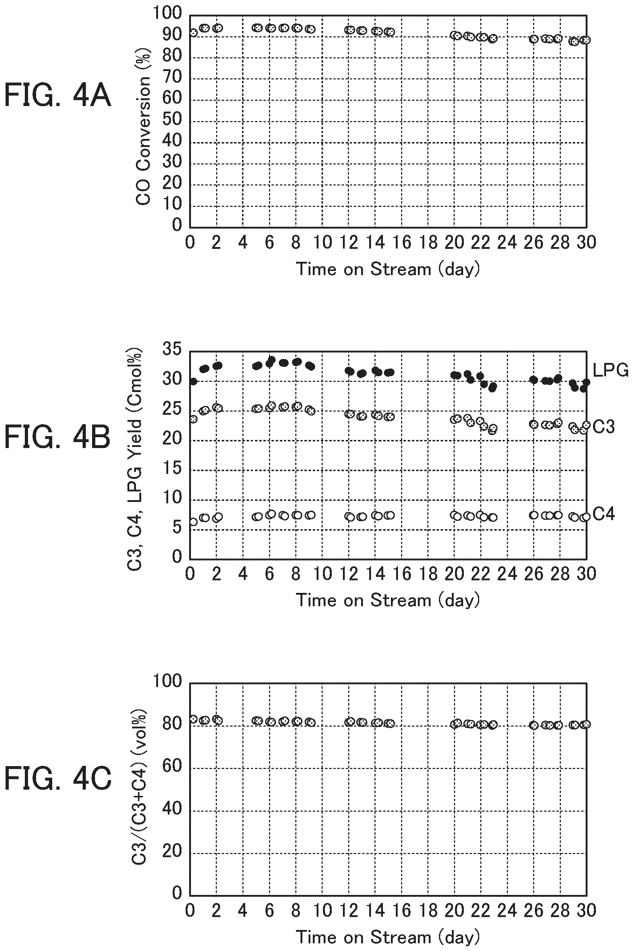

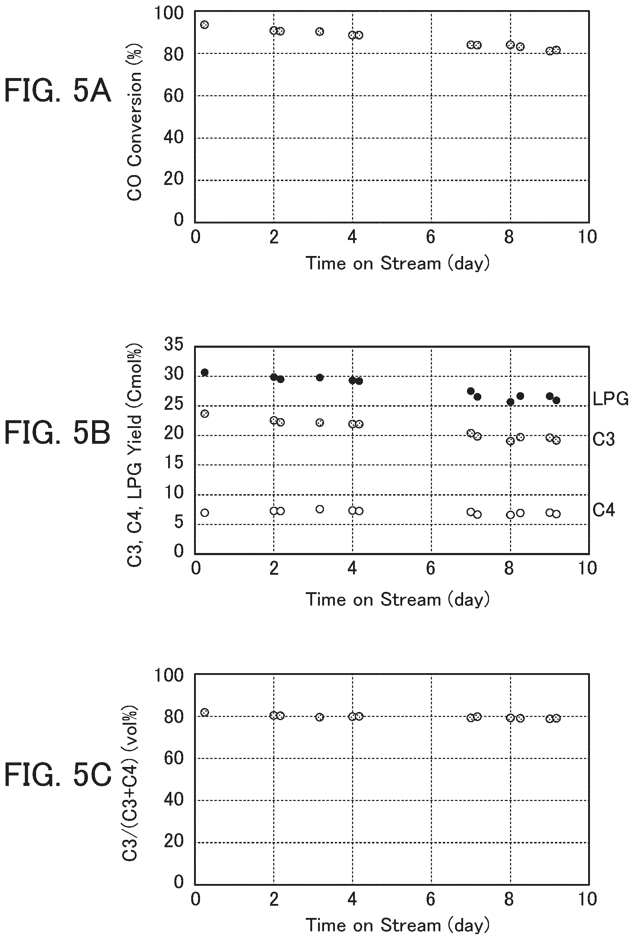

As illustrated in Tables 1 to 4 and Figs. 4 and 5, as the ratio (M1/(M1+M2)) increased, the mid-term and long-term performance of the catalyst for synthesizing liquefied petroleum gas improved. In particular, the catalysts for synthesizing liquefied petroleum gas of Examples 7 to 12 were found to be able to produce propane stably over a long period of time, resisting deactivation at around 320°C.

## Claims

1. A catalyst for synthesizing liquefied petroleum gas, the catalyst comprising:
a Cu-Zn-based catalytic material; and
an MFI-type zeolite catalytic material supporting Pt,
wherein a ratio (M1/(M1+M2)) of mass (M1) of the Cu-Zn-based catalytic material to total mass of the mass (M1) of the Cu-Zn-based catalytic material and mass (M2) of the MFI-type zeolite catalytic material is 0.30 or more and 0.95 or less.

2. The catalyst for synthesizing liquefied petroleum gas according to claim 1, wherein the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material is 0.30 or more and less than 0.70.

3. The catalyst for synthesizing liquefied petroleum gas according to claim 1, wherein the ratio (M1/(M1+M2)) of the mass (M1) of the Cu-Zn-based catalytic material to the total mass of the mass (M1) of the Cu-Zn-based catalytic material and the mass (M2) of the MFI-type zeolite catalytic material is 0.50 or more and 0.95 or less.

4. The catalyst for synthesizing liquefied petroleum gas according to any one of claims 1 to 3, wherein the MFI-type zeolite catalytic material supports only Pt.

5. The catalyst for synthesizing liquefied petroleum gas according to any one of claims 1 to 3, wherein the MFI-type zeolite catalytic material further supports Pd.

6. The catalyst for synthesizing liquefied petroleum gas according to claim 5, wherein a ratio (M_{Pd}/(M_{Pt}+M_{Pd})) of mass (M_{Pd}) of Pd to total mass (M_{Pt}+M_{Pd}) of mass (M_{Pt}) of Pt and the mass (M_{Pd}) of Pd supported on the MFI-type zeolite catalytic material is 0.70 or less.

7. The catalyst for synthesizing liquefied petroleum gas according to claim 5 or 6, wherein a ratio of the total mass (M_{Pt}+M_{Pd}) of the mass (M_{Pt}) of Pt and the mass (M_{Pd}) of Pd in the MFI-type zeolite catalytic material to the mass (M2) of the MFI-type zeolite catalytic material is 0.1 mass% or more and 1.0 mass% or less.

8. The catalyst for synthesizing liquefied petroleum gas according to any one of claims 1 to 7, wherein
the Cu-Zn-based catalytic material and the MFI-type zeolite catalytic material exist independently from each other, and
both of the Cu-Zn-based catalytic material and the MFI-type zeolite catalytic material are in the form of granulated powder or molded body.

9. A method for producing liquefied petroleum gas, the method comprising:
a reduction treatment step of reducing the catalyst for synthesizing liquefied petroleum gas according to any one of claims 1 to 8;
a supply step of supplying carbon monoxide and hydrogen to the catalyst for synthesizing liquefied petroleum gas reduced in the reduction treatment step; and
a synthesis step of synthesizing liquefied petroleum gas by reacting the carbon monoxide and the hydrogen supplied in the supply step using the catalyst for synthesizing liquefied petroleum gas to be reduced.

10. The method for producing liquefied petroleum gas according to claim 9, wherein a gas hourly space velocity (GHSV) for supplying the carbon monoxide and the hydrogen is 300/h or more and 20000/h or less in the supply step.

11. The method for producing liquefied petroleum gas according to claim 9 or 10, wherein the carbon monoxide and the hydrogen are reacted at a temperature of 260°C or more and 360°C or less in the synthesis step.

12. The method for producing liquefied petroleum gas according to any one of claims 9 to 11, wherein the carbon monoxide and the hydrogen are reacted under a pressure of 2.0 MPa or more and 6.0 MPa or less in the synthesis step.
